# EUROPEAN PATENT SPECIFICATION

(11) **EP 2 432 396 B1**
(45) Date of publication and mention of the grant of the patent: **09.08.2017**
(21) Application number: 10727491.2
(22) Date of filing: 21.05.2010
(51) Int. Cl.: A61B 5/20

(54) **METHOD AND APPARATUS FOR MEASURING BLADDER PRESSURE**
VERFAHREN UND VORRICHTUNG FÜR BLASENDRUCKMESSUNG
PROCÉDÉ ET APPAREIL POUR MESURER LA PRESSION DE LA VESSIE

(30) Priority: 21.05.2009 GB 0908766
(43) Date of publication of application: 28.03.2012
(73) Proprietor: Newcastle-Upon-Tyne Hospitals NHS Trust, Newcastle-upon-Tyne Tyne and Wear NE7 7DN (GB)
(72) Inventor: CLARKSON, Becky, Newcastle-upon-Tyne Tyne&Wear NE7 7PQ (GB); DRINNAN, Michael, Alnwick Northumberland NE66 1QY (GB); GRIFFITHS, Clive, Javan, Newcastle-upon-Tyne Tyne&Wear NE7 7RA (GB)
(74) Representative: Byworth, Ian James
(86) International application number: PCT/GB2010/050829
(87) International publication number: WO 2010/133887

(56) References cited:
- WO-A1-00/27285
- US-A- 5 807 278
- US-A1- 2004 260 163
- BLAKE ET AL: "Noninvasive Techniques for the Measurement of Isovolumetric Bladder Pressure" JOURNAL OF UROLOGY, LIPPINCOTT WILLIAMS & WILKINS, BALTIMORE, MD, US LNKD- DOI:10.1097/01.JU.0000102685.44036.B9, vol. 171, no. 1, 1 January 2004 (2004-01-01), pages 12-19, XP005532179 ISSN: 0022-5347

## Description

The present invention relates to a method and an apparatus for carrying out measurements of bladder pressure in male patients in order to facilitate the diagnosis of urinary tract obstructions and impaired bladder contraction.

It is known to make continuous bladder pressure measurements in men using invasive techniques which involve the introduction of a catheter through the patient's urethra and into their bladder. Although this procedure is normally carried out under aseptic conditions, it is common for bacteria from the skin and urethral passage to be introduced into the bladder, which can lead to infection. Moreover, the introduction of a catheter into this area, which is very sensitive, is very uncomfortable for the patient and can sometimes cause bleeding.

It is also known to use non invasive techniques to make bladder pressure measurements in an attempt to mitigate the disadvantages of invasive techniques such as those described above. For example, European patent publication number EP1124486 discloses a method and apparatus for carrying out urodynamic investigations using a pneumatic cuff. The cuff is placed around the penis, and after commencement of urine flow through the penis, it is inflated over a period of time in order to occlude the urethra before voiding (that is, emptying of the bladder) is complete. The relationship between urine flow rate and the cuff pressure is used to determine a measurement of the isovolumetric bladder pressure for the patient (i.e. the pressure in the patient's bladder when the urine flow rate is zero).

By way of further example, United States patent publication number 5807278 discloses a method and apparatus for carrying out non-invasive bladder pressure and urine flow measurements using a pneumatic cuff. The cuff is placed around the penis, and is inflated in order to occlude the urethra and prevent the flow of urine. Upon the urge to void, the cuff is gradually deflated whilst the bladder muscle contracts. When the pressure generated by the bladder is equal to that exerted on the urethra by the cuff, then urine flow is initiated through the urethra. The cuff is then rapidly deflated in order to allow urine to flow freely under the force of the bladder of the patient, and the bladder pressure is ascertained.

Despite being a useful means of assessing the health of the urinary tract of the patient, the above disclosed methods and apparatuses can only provide the clinician with a very limited number of samples (for example, one, two or three) during one particular void and as such do not provide the clinician with a continuous measurement of bladder pressures throughout the void.

An aim of the present invention is to provide a method and apparatus for measuring bladder pressure, which overcomes or at least alleviates the problems associated with known techniques.

In accordance with a first aspect of the present invention there is provided a method for measuring bladder pressure in a patient, comprising the steps of: -
(i) attaching an inflatable cuff around the penis of the patient;
(ii) maintaining a flow of urine at a predetermined flow rate for a period of time during voiding of the bladder, by means of deflating or inflating the cuff over said period of time, as required; and
(iii) measuring the cuff pressures required to maintain said predetermined urine flow rate during said period of time.

This provides the advantage that the bladder pressure can be measured continuously during the void. It is to be appreciated that the term "continuous" is to be construed as meaning providing bladder pressure which can be sampled at arbitrarily narrow intervals, during a single void of the bladder. Typically, there are around 10 samples of bladder pressure taken per second during a single void.

Preferably, the step of maintaining said predetermined urine flow rate for said period of time may comprise measuring the actual urine flow rate and inflating or deflating the cuff as required in order that the urine flow rate is substantially equal to said predetermined flow rate.

Preferably, the actual urine flow rate may be measured by means of creating electrical signals representative of the actual urine flow rate.

Preferably, the method may further comprise determining bladder pressure measurements during said period of time based upon the measured cuff pressures required to maintain said predetermined urine flow rate during said period of time.

The method may comprise inflating the cuff in order to occlude the urethra, before commencement of urine flow through the penis and after the cuff has been attached around the penis of the patient, and then deflating the cuff until the urine flow reaches said predetermined urine flow rate.

In this way, the cuff is pre-inflated. It is to be appreciated that the term "occlude" is to be construed as meaning applying sufficient pressure to the urethra using the cuff to substantially prevent the flow of urine through the penis.

This provides the advantage that there is no initial surge of urine and as such there is more time available during the void for the clinician to make bladder pressure measurements.

Alternatively, the method may comprise inflating the cuff after commencement of urine flow through the penis and after the cuff has been attached around the penis of the patient, as required, until the urine flow reaches said predetermined urine flow rate.

This provides the advantage that the inflation of the cuff can be triggered automatically as soon as the flow of urine is detected. As such, there is no requirement for either the clinician or the patient to make any judgements regarding when the patient feels the urge to void.

Preferably however, the method may comprise partially inflating the cuff before commencement of urine flow through the penis and after the cuff has been attached around the penis of the patient, and then inflating or deflating the cuff as required, until the urine flow reaches said predetermined flow rate.

It is to be appreciated that the term "partially inflating the cuff" is to be construed as meaning applying pressure to the penis to an extent that the flow of urine through the penis is not completely prevented. In this way, the patient is able to void past the pressure applied.

The partial inflation of the cuff before commencement of voiding (in other words, partial pre-inflation) provides the advantage that the "dead time" is reduced. It is to be appreciated that the dead time is the time required to inflate the cuff until it exerts pressure on the penis. For example, in the event that the patient begins to void, and the clinician wishes to inflate the cuff, then it takes a certain amount of time for the cuff to inflate from its completely deflated state to the extent required by the clinician. However, if the cuff is partially inflated before commencement of voiding, then it takes less time to inflate the cuff to that extent required by the clinician, thereby increasing the proportion of the void time during which measurements can be made.

The partial inflation of the cuff before commencement of voiding (in other words, partial pre-inflation) provides the further advantage that the further inflation of the cuff can be triggered automatically as soon as the flow of urine is detected. As such, there is no requirement for either the clinician or the patient to make any judgements regarding when the patient feels the urge to void.

In accordance with a second aspect of the present invention there is provided an apparatus for measuring bladder pressure in a patient, comprising: -
(i) an inflatable cuff for attachment around the penis of the patient;
(ii) a pressure generating device for inflating the cuff and deflating the cuff, as required;
(iii) a control means for maintaining a flow of urine at a predetermined flow rate for a period of time during voiding of the bladder, by means of deflating or inflating the cuff over said period of time, as required; and
(iv) a measuring means for measuring the cuff pressures required to maintain said predetermined urine flow rate during said period of time.

Preferably, said apparatus further comprises flow measurement means for measuring the actual urine flow rate and inputting the measured flow rate to said control means.

Preferably, said flow measurement means creates electrical signals representative of the actual urine flow rate.

Preferably, the apparatus further comprises analysis means for determining bladder pressure measurements during said period of time based upon the measured cuff pressures required to maintain said predetermined urine flow rate during said period of time.

Preferred embodiments of the present invention will now be described, by way of example only and not in any limitative sense, with reference to the accompanying drawings in which: -
Figure 1 shows a block diagram of an apparatus in accordance with a first embodiment of the present invention;
Figure 2 shows a block diagram of an apparatus in accordance with a second embodiment of the present invention;
Figure 3 shows a block diagram of an apparatus in accordance with a third embodiment of the present invention;
Figures 4a to 4f show examples of cuff pressure measurements over time for different patients;
Figure 5 shows a flow chart of an embodiment of a method in accordance with the present invention; and
Figure 6 is a schematic diagram illustrating the method of selecting the period of time during which the pressure measurements taken by the clinician can be assumed to be an accurate measurement of the bladder pressure of the patient.

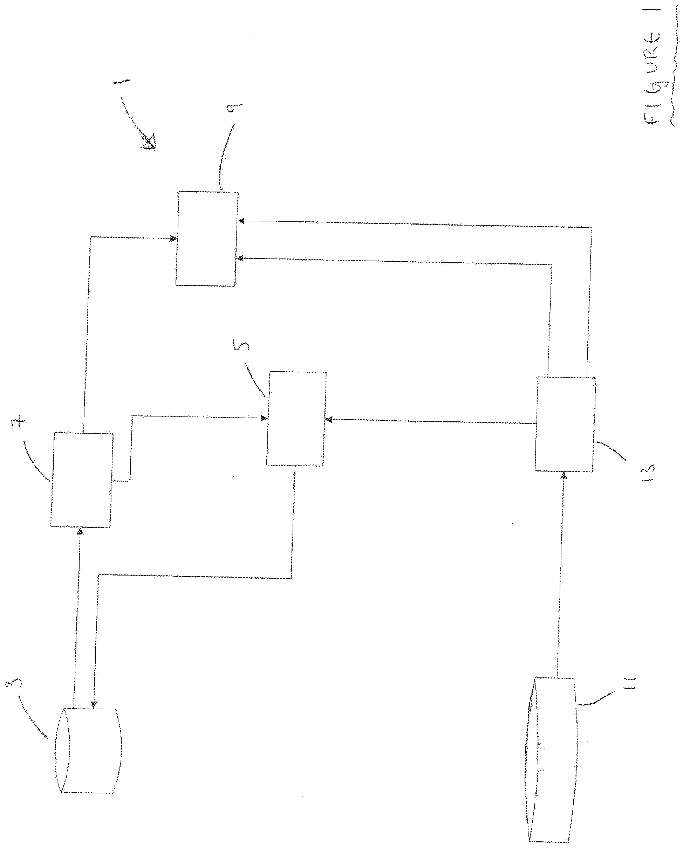

Referring now to Figure 1, an apparatus for measuring bladder pressure in a patient is represented generally by reference numeral 1.

The apparatus 1 comprises an inflatable penile cuff 3 which is adapted to be secured around the patient's penis. The cuff 3 is operatively connected to a pressure control system 5 incorporating a pneumatic pump, which inflates the cuff 3 and deflates the cuff 3 as required, which will be explained in further detail below.

The apparatus 1 further comprises a pressure measurement device 7 which is operatively connected to the cuff 3 and which directly measures the pressure applied to the penis by the cuff 3. The pressure measurement device 7 is operatively connected to a recording and analysis system 9, which records the cuff pressure measured by the measurement device 7.

The apparatus 1 further comprises a flow sensor 11, which converts the force applied by the urine collected during voiding into an electrical signal which is then relayed to a flow measurement device 13. In this embodiment, the flow sensor is a load cell, but it is to be appreciated that any other suitable flow sensor could be used, for example a spinning disk. The flow measurement device 13 uses the electrical signal received from the load cell 11 and establishes the flow rate and the volume of urine voided from the patient's bladder. These measurements of flow rate and voided volume are inputted into the recording and analysis system 9, along with the cuff pressure as previously described.

The recording and analysis system 9 uses the measurements of flow rate, voided volume and cuff pressure to provide the clinician with a plot of bladder pressure over time, for a particular flow rate.

In order to maintain the urine flow rate and further, in order to maintain that flow rate at a low level, a feedback mechanism is employed between the flow measurement device 13 and the cuff 3, with the cuff 3 being inflated and deflated as required, in order to maintain the urine flow at a constant low rate. To elaborate, the pressure control system is set to a predetermined flow rate, for example, 2.5mls⁻¹. This could be achieved either by pre-programming of the pressure control system or by the clinician manually inputting the value of the pre-determined flow rate. As the patient's bladder voids, the load cell 11 provides the flow measurement device 13 with an electrical signal which provides a measurement of flow rate at a particular point in time. This measured value of the flow rate is inputted into the pressure control system 5, which compares it with the predetermined flow rate. If the measured value of the flow rate is different from the predetermined flow rate, then the pressure control system 5 either inflates or deflates the cuff 3, as required, until the subsequently measured value of the flow rate changes sufficiently so that it is equal to the predetermined flow rate.

It is however, also to be appreciated that the clinician could instead manually input a predetermined flow rate into the pressure control system 5, as opposed to the pressure control system 5 being pre-programmed with the predetermined flow rate.

It is also to be appreciated that the predetermined flow rate of 2.5mls⁻¹ is provided by means of example only, and that any suitable predetermined flow rate (that is, a predetermined flow rate which is lower than the natural urine flow rate of the patient), could alternatively be used.

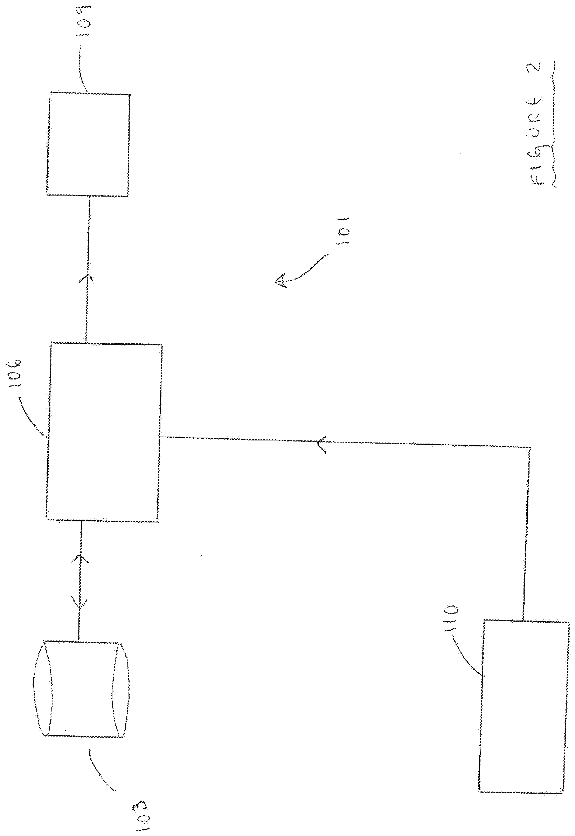

Referring now to Figure 2, an apparatus for measuring bladder pressure in a patient is represented generally by reference numeral 101.

The apparatus 101 comprises an inflatable penile cuff 103 which is adapted to be secured around the patient's penis. The cuff 103 is operatively connected to a pressure measurement and control system 106 incorporating a pneumatic pump, which inflates the cuff 103 and deflates the cuff 103 as required, which will be explained in further detail below. The pressure measurement and control system 106 further comprises a pressure measurement device which is operatively connected to the cuff 103 and which directly measures the pressure applied to the penis by the cuff 103. The pressure measurement and control system 106 is operatively connected to a recording and analysis system 109, which records the cuff pressure measured by the pressure measurement and control system 106.

The apparatus 101 further comprises a flow measurement device 110, which converts the force applied by the urine collected during voiding into an electrical signal and establishes the flow rate of urine voided from the patient's bladder. These measurements of flow rate are then inputted into the pressure measurement and control system 106, which maintains the flow rate at the predetermined value and further, maintains that flow rate at a low level using a feedback mechanism. To elaborate, the pressure measurement and control system 106 is set with a predetermined flow rate, for example, 2.5mls⁻¹. As the patient's bladder voids, the flow measurement device 110 provides a measurement of flow rate at a particular point in time. This measured value of the flow rate is inputted into the pressure measurement and control system 106, which compares it with the inputted predetermined flow rate. If the measured value of the flow rate is different from the predetermined flow rate, then the pressure measurement and control system 106 either inflates or deflates the cuff 103, as required, until the subsequently measured value of the flow rate changes sufficiently so that it is equal to the predetermined flow rate.

The recording and analysis system 109 records the measurements of cuff pressure obtained by the pressure measurement device of the pressure measurement and control system 106 required to maintain the predetermined flow rate, in order to provide the clinician with a plot of bladder pressure over time, for the particular predetermined value of the flow rate.

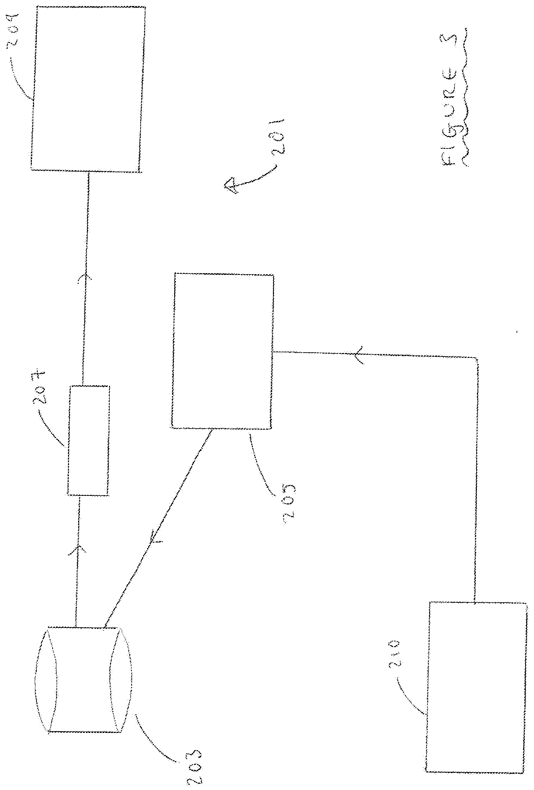

Referring now to Figure 3, an apparatus for measuring bladder pressure in a patient is represented generally by reference numeral 201.

The apparatus 201 comprises an inflatable penile cuff 203 which is adapted to be secured around the patient's penis. The cuff 203 is operatively connected to a pressure measurement device 207 and a pressure control system 205 incorporating a pneumatic pump. The pressure measurement device 207 directly measures the pressure applied to the penis by the cuff 203. The pressure control system 205 inflates the cuff 203 and deflates the cuff 203 as required, which will be explained in further detail below. The pressure measurement device 207 is operatively connected to a recording and analysis system 209, which records the cuff pressure measured by the pressure measurement device 207.

The apparatus 201 further comprises a flow measurement device 210, which converts the force applied by the urine collected during voiding into an electrical signal and establishes the flow rate of urine voided from the patient's bladder. These measurements of flow rate are then inputted into the pressure control system 205, which maintains the flow rate at the predetermined value and further, maintains that flow rate at a low level using a feedback mechanism. To elaborate, the pressure control system 205 is set with a predetermined flow rate, for example, 2.5mls⁻¹. As the patient's bladder voids, the flow measurement device 210 provides a measurement of urine flow rate at a particular point in time. This measured value of the urine flow rate is inputted into the pressure control system 205, which compares it with the inputted predetermined flow rate. If the measured value of the flow rate is different from the predetermined flow rate, then the pressure control system 205 inflates or deflates the cuff 203, as required, until the subsequently measured value of the flow rate changes sufficiently so that it is equal to the predetermined flow rate.

The recording and analysis system 209 records the measurements of cuff pressure obtained by the pressure measurement device 207 required to maintain the predetermined flow rate, in order to provide the clinician with a plot of bladder pressure over time, for the particular predetermined value of the flow rate.

It is to be appreciated that an upper limit of cuff pressure may be set, in order to prevent injury to the patient.

### Procedure - Example 1 (Pre-inflation)

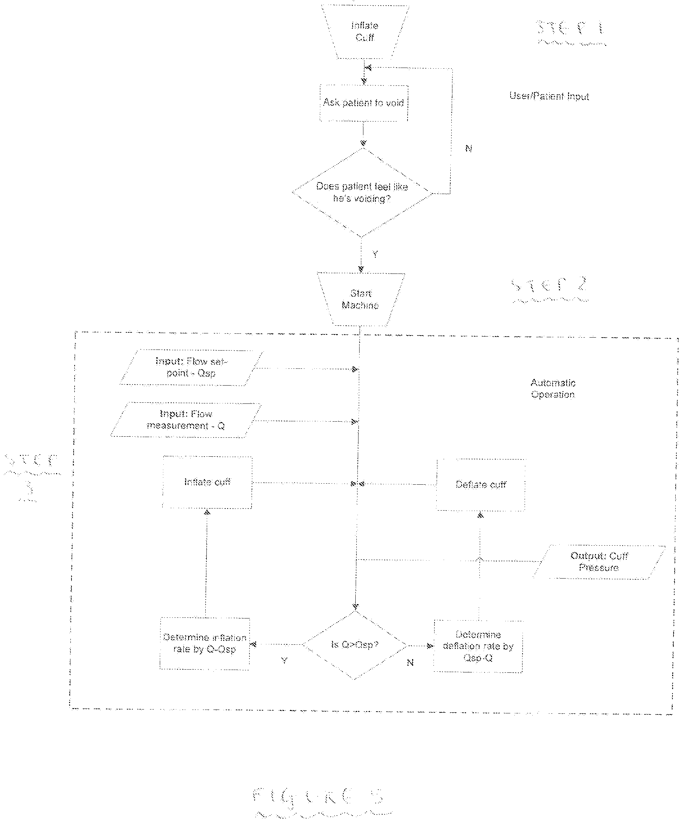

Referring now to Figure 5, a flow chart is shown which illustrates this procedural example.

In the event that a clinician wishes to measure the bladder pressure of a patient, an inflatable cuff is placed around the patient's penis and is pre-inflated to 200 cmH₂0 using a pneumatic pump (Step 1). The patient is asked when he feels that the cuff is physically stopping his flow of urine, that is, he is asked to identify when he thinks he would be voiding were it not for the presence of the cuff. At this point, the bladder is trying to empty and the physical obstruction of the cuff is stopping the flow, and so the apparatus is activated (Step 2). The method of the invention is then performed, the cuff being deflated until a predetermined urine flow rate is detected by the flow measurement device. The pressure applied by the cuff is then modulated by means of deflation or inflation of the cuff as required, in order to maintain the urine flow rate at the predetermined value (Step 3). For example, if the urine flow rate falls below the predetermined value (that is, Qₛₚ is greater than Q), then the cuff is deflated until the flow rate reaches the predetermined value. Conversely, if the urine flow rate increases above the predetermined value (that is, Qₛₚ is less than Q), then the cuff is inflated until the flow rate is substantially equal to the predetermined value once more.

In this way, the flow rate of urine is allowed to continue but at a low, predetermined value (for example 2.5mls⁻¹), using the cuff as a means of controlling the urine flow rate and a means of ensuring that for as long as possible during the voiding of the patient's bladder, the urine flow rate is maintained at the predetermined value.

### Procedure - Example 2 (No pre-inflation)

In the event that a clinician wishes to measure the bladder pressure of a patient, the inflatable cuff is placed around the patient's penis. Soon after the patient begins to void, the cuff is inflated using the pneumatic pump until a predetermined urine flow rate is detected by the flow measurement device. The pressure applied by the cuff is then modulated by means of deflation or inflation of the cuff as required, in order to maintain the urine flow rate at the predetermined value. For example, if the urine flow rate falls below the predetermined value, then the cuff is deflated until the flow rate reaches the predetermined value. Conversely, if the urine flow rate increases above the predetermined value, then the cuff is inflated until the flow rate is substantially equal to the predetermined value once more.

In this way, the flow rate of urine is allowed to continue but at a low, predetermined value (for example 2.5mls⁻¹), using the cuff as a means of controlling the urine flow rate and a means of ensuring that for as long as possible during the voiding of the patient's bladder, the urine flow rate is maintained at the predetermined value.

### Procedure - Example 3 (Partial pre-inflation)

In the event that a clinician wishes to measure the bladder pressure of a patient, an inflatable cuff is placed around the patient's penis and is pre-inflated using a pneumatic pump. In this example, the cuff is only partially inflated, as opposed to being fully inflated in order to completely occlude the urethra, as is the case with Example 1. With this amount of pressure applied, upon feeling the urge to void, the patient can manage to commence voiding, and the apparatus is activated. At this point, the pressure applied by the cuff is modulated by means of deflation or inflation of the cuff as required, until the measured urine flow rate reaches the predetermined value. The urine flow rate is then maintained at the predetermined value. For example, if the urine flow rate falls below the predetermined value, then the cuff is deflated until the flow rate reaches the predetermined value. Conversely, if the urine flow rate increases above the predetermined value then the cuff is inflated until the flow rate is substantially equal to the predetermined value once more.

In this way, the flow rate of urine is allowed to continue but at a low, predetermined value (for example 2.5mls⁻¹), using the cuff as a means of controlling the urine flow rate and a means of ensuring that for as long as possible during the voiding of the patient's bladder, the urine flow rate is maintained at the predetermined value.

The reason why the urine flow rate is maintained at a *low* rate in all of these examples is so that the change in the filled volume of the bladder over time is small. A low urine flow rate also ensures that the pressure drop across any resistance to flow between the bladder and the patient's penis is kept to a minimum. Moreover, in the event that there is a flow of urine, a proportion of the pressure generated by the bladder is used to produce that flow, and the cuff would then measure the residual pressure. If the urine flow is maintained at a low rate then the residual pressure measured would be closer to the bladder pressure. In this way, the pressure applied by the cuff provides a very good approximation of the actual bladder pressure and the pressure changes in the cuff can accurately reflect the pressure changes in the bladder.

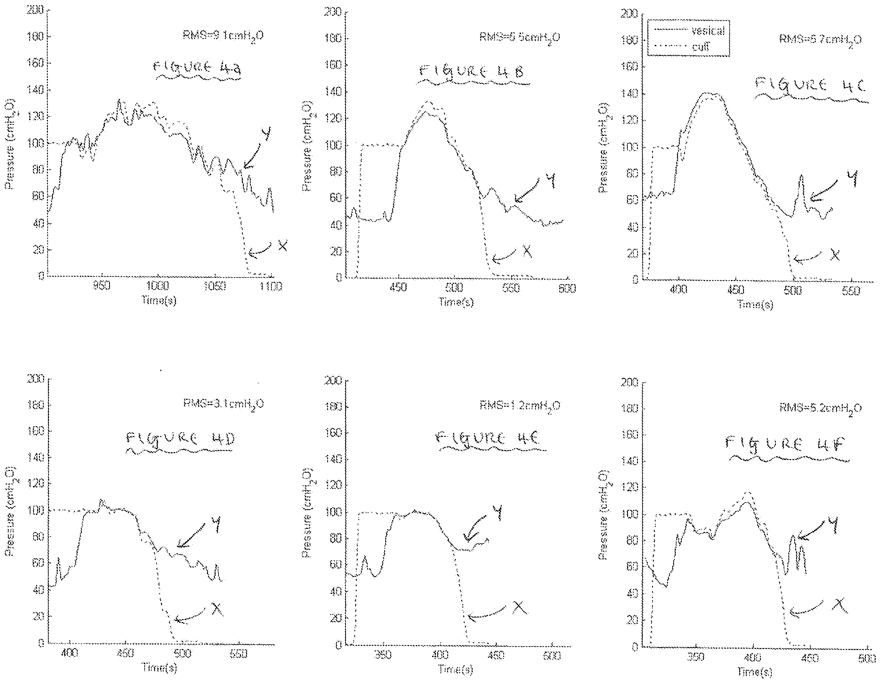

The inventors have shown that the bladder pressures measured using the method of the present invention are very close to those bladder pressures measured simultaneously using invasive but nevertheless very accurate methods such as catheterisation, as shown by the graphs of Figures 4a to 4f, which will be discussed below.

With reference to Figures 4a to 4f, each graph provides results for a particular patient, during one particular void. For example, Figure 4a shows a plot X of the values of applied cuff pressure measured over time using the method of the present invention, whilst maintaining the flow rate at a constant, low predetermined flow rate. Figure 4a also shows a second plot Y of the values of bladder pressure measured simultaneously over time invasively using a catheterisation method, during the same void in a patient. It can be seen that for a large proportion of the void, that is, at those times when the measured flow rate is substantially the same as the predetermined value of the flow rate, the measured value of applied cuff pressure is the same as the bladder pressure measured invasively using a catheterisation method.

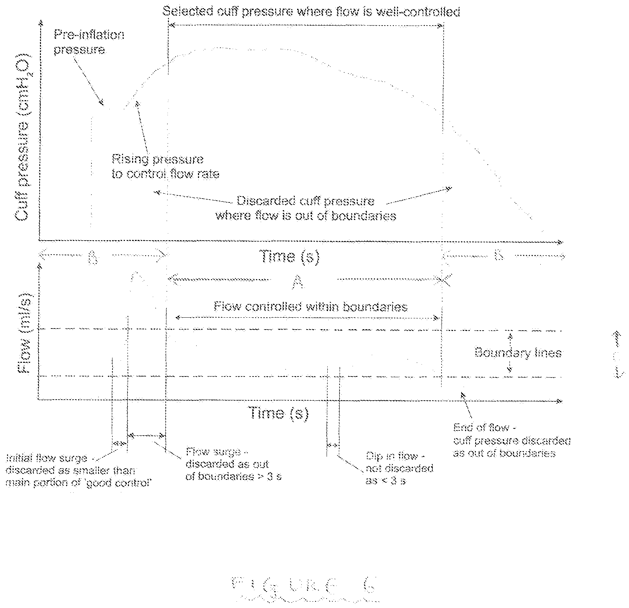

With reference to Figure 6, an algorithm was developed, which assesses at which times during one particular void the value of the cuff pressure measured using the method of the present invention is an accurate estimate of the bladder pressure.

The way in which this is achieved is by continually comparing the measured flow rate to the predetermined value of the flow rate. When the two are substantially equal to each other, or when the measured flow rate is within a certain range either side of the predetermined value of the flow rate (represented by flow rate range C on Figure 6), the measured cuff pressure can be assumed to be an accurate estimate of the bladder pressure. This period of time is represented by the period A in Figure 6. The algorithm discounts those cuff pressures measured when the measured flow rate is either not substantially equal to the predetermined value of the flow rate or is not within flow rate range C, since the cuff pressures measured at that time will not be an accurate estimate of the bladder pressure. These periods of time are represented by the period B in Figure 6.

In this way, the algorithm generates a data set including "good" and "bad" measurements of bladder pressure. The algorithm selects "good" data by comparing the actual measured flow rate with the predetermined value of the flow rate, and if they are substantially equal or within the flow rate range C, then the measured value of the cuff pressure at that point is "good" data and can be assumed to be an accurate estimate of the bladder pressure.

The present invention, several embodiments of which are described above, can provide information for use by a clinician for investigating and comparing healthy bladders, diagnosing bladder outlet obstruction and impaired bladder contraction in patients.

It will be appreciated by persons skilled in the art that the above embodiments have been described by way of example only, and not in any limitative sense, and that various alterations and modifications are possible without departing from the scope of the invention as defined by the appended claims.

## Claims

1. A method for measuring bladder pressure in a patient, comprising the step of: -
(i) attaching an inflatable cuff (3) around the penis of the patient, **characterised by** comprising the further steps of
(ii) maintaining a flow of urine at a predetermined flow rate for a period of time during voiding of the bladder, by means of deflating or inflating the cuff (3) over said period of time, as required; and
(iii) measuring the cuff pressures required to maintain said predetermined urine flow rate during said period of time.

2. A method for measuring bladder pressure in a patient as claimed in claim 1, wherein the step of maintaining said predetermined urine flow rate for said period of time comprises measuring the actual urine flow rate and inflating or deflating the cuff (3) as required in order that the urine flow rate is substantially equal to said predetermined flow rate.

3. A method for measuring bladder pressure in a patient as claimed in claim 2, wherein the actual urine flow rate is measured by means of creating electrical signals representative of the actual urine flow rate.

4. A method for measuring bladder pressure in a patient as claimed in any one of the previous claims, further comprising determining bladder pressure measurements during said period of time based upon the measured cuff pressures required to maintain said predetermined urine flow rate during said period of time.

5. A method for measuring bladder pressure in a patient as claimed in any one of the previous claims, comprising inflating the cuff (3) in order to occlude the urethra, before commencement of urine flow through the penis and after the cuff (3) has been attached around the penis of the patient, and then deflating the cuff (3) until the urine flow reaches said predetermined urine flow rate.

6. A method for measuring bladder pressure in a patient as claimed in any one of claims 1 to 4, comprising partially inflating the cuff (3) before commencement of urine flow through the penis and after the cuff (3) has been attached around the penis of the patient, and then inflating or deflating the cuff (3) as required, until the urine flow reaches said predetermined flow rate.

7. A method for measuring bladder pressure in a patient as claimed in any one of claims 1 to 4, comprising inflating the cuff (3) after commencement of urine flow through the penis and after the cuff (3) has been attached around the penis of the patient, as required, until the urine flow reaches said predetermined urine flow rate.

8. An apparatus (1) for measuring bladder pressure in a patient, comprising: -
(i) an inflatable cuff (3) for attachment around the penis of the patient;
(ii) a pressure generating device for inflating the cuff (3) and deflating the cuff (3), as required; **characterized by**
(iii) a control means (5) for maintaining a flow of urine at a predetermined flow rate for a period of time during voiding of the bladder, by means of deflating or inflating the cuff (3) over said period of time, as required; and
(iv) a measuring means (7) for measuring the cuff pressures required to maintain said predetermined urine flow rate during said period of time.

9. An apparatus (1) for measuring bladder pressure in a patient as claimed in claim 8, further comprising flow measurement means (13) for measuring the actual urine flow rate and inputting the measured flow rate to said control means (5).

10. An apparatus (1) for measuring bladder pressure in a patient as claimed in claim 9, wherein said flow measurement means (13) creates electrical signals representative of the actual urine flow rate.

11. An apparatus (1) for measuring bladder pressure in a patient as claimed in any one of claims 8 to 10, further comprising analysis means (9) for determining bladder pressure measurements during said period of time based upon the measured cuff pressures required to maintain said predetermined urine flow rate during said period of time.

## Patentansprüche

1. Verfahren zur Messung des Blasendrucks bei einem Patienten, umfassend die folgenden Schritte:
(i) Anbringen einer aufblasbaren Manschette (3) um den Penis des Patienten, **dadurch gekennzeichnet, dass** es ferner die folgenden Schritte umfasst:
(ii) Halten eines Flusses von Harn bei einer vorbestimmten Flussrate für eine Zeitdauer während des Entleerens der Blase durch Deflatieren oder Inflatieren der Manschette (3) nach Bedarf über die Zeitdauer; und
(iii) Messen der Manschettendrücke, die zum Halten der vorbestimmten Harnflussrate während der Zeitdauer erforderlich sind.

2. Verfahren zur Messung des Blasendrucks bei einem Patienten nach Anspruch 1, wobei der Schritt des Haltens der vorbestimmten Harnflussrate für die Zeitdauer ein Messen der tatsächlichen Harnflussrate und Inflatieren oder Deflatieren der Manschette (3) nach Bedarf umfasst, damit die Harnflussrate im Wesentlichen der vorbestimmten Flussrate entspricht.

3. Verfahren zur Messung des Blasendrucks bei einem Patienten nach Anspruch 2, wobei die tatsächliche Harnflussrate durch Erzeugen elektrischer Signale, welche die tatsächliche Harnflussrate darstellen, gemessen wird.

4. Verfahren zur Messung des Blasendrucks bei einem Patienten nach einem der vorhergehenden Ansprüche, ferner umfassend ein Bestimmen von Blasendruckmessungen während der Zeitdauer basierend auf den gemessenen Manschettendrücken, die zum Halten der vorbestimmten Harnflussrate während der Zeitdauer erforderlich sind.

5. Verfahren zur Messung des Blasendrucks bei einem Patienten nach einem der vorhergehenden Ansprüche, umfassend ein Inflatieren der Manschette (3) vor Beginn von Harnfluss durch den Penis und nach dem Anbringen der Manschette (3) um den Penis des Patienten, um die Harnröhre zu okkludieren, und anschließendes Deflatieren der Manschette (3), bis der Harnfluss die vorbestimmte Harnflussrate erreicht.

6. Verfahren zur Messung des Blasendrucks bei einem Patienten nach einem Ansprüche 1 bis 4, umfassend ein teilweises Inflatieren der Manschette (3) vor Beginn von Harnfluss durch den Penis und nach dem Anbringen der Manschette (3) um den Penis des Patienten und anschließendes Inflatieren oder Deflatieren der Manschette (3) nach Bedarf, bis der Harnfluss die vorbestimmte Harnflussrate erreicht.

7. Verfahren zur Messung des Blasendrucks bei einem Patienten nach einem Ansprüche 1 bis 4, umfassend ein Inflatieren der Manschette (3) nach Beginn von Harnfluss durch den Penis und nach dem Anbringen der Manschette (3) um den Penis des Patienten nach Bedarf, bis der Harnfluss die vorbestimmte Harnflussrate erreicht.

8. Vorrichtung (1) zum Messen des Blasendrucks bei einem Patienten, umfassend:
(i) eine aufblasbare Manschette (3) zur Anbringung um den Penis des Patienten;
(ii) eine Druckerzeugungseinrichtung zum Inflatieren der Manschette (3) und Deflatieren der Manschette (3) nach Bedarf;
**gekennzeichnet durch**
(iii) ein Steuermittel (5) zum Halten eines Flusses von Harn bei einer vorbestimmten Flussrate für eine Zeitdauer während des Entleerens der Blase durch Deflatieren oder Inflatieren der Manschette (3) nach Bedarf über die Zeitdauer; und
(iv) ein Messmittel (7) zum Messen der Manschettendrücke, die zum Halten der vorbestimmten Harnflussrate während der Zeitdauer erforderlich sind.

9. Vorrichtung (1) zum Messen des Blasendrucks bei einem Patienten nach Anspruch 8, ferner umfassend Flussmessmittel (13) zum Messen der tatsächlichen Harnflussrate und Eingeben der gemessenen Flussrate in das Steuermittel (5).

10. Vorrichtung (1) zum Messen des Blasendrucks bei einem Patienten nach Anspruch 9, wobei das Flussmessmittel (13) elektrische Signale erzeugt, welche die tatsächliche Harnflussrate darstellen.

11. Vorrichtung (1) zum Messen des Blasendrucks bei einem Patienten nach einem der Ansprüche 8 bis 10, ferner umfassend Analysemittel (9) zum Bestimmen Blasendruckmessungen während der Zeitdauer basierend auf den gemessenen Manschettendrücken, die zum Halten der vorbestimmten Harnflussrate während der Zeitdauer erforderlich sind.

## Revendications

1. Procédé de mesure de la pression de la vessie chez un patient, comprenant l'étape de :
(i) attachement d'un brassard gonflable (3) autour du pénis du patient, caractérisé comme comprenant les autres étapes de
(ii) maintien d'un écoulement d'urine à un débit prédéterminé pour une période de temps durant le vidage de la vessie, au moyen du dégonflement ou du gonflement du brassard (3) sur ladite période de temps, comme nécessaire ; et
(iii) mesure des pressions du brassard requises pour maintenir ledit débit d'urine prédéterminé durant ladite période de temps.

2. Procédé de mesure de la pression de la vessie chez un patient selon la revendication 1, dans lequel l'étape de maintien dudit débit d'urine prédéterminé durant ladite période de temps comprend la mesure du débit d'urine réel et le gonflement ou le dégonflement du brassard (3) comme nécessaire pour que le débit d'urine soit sensiblement égal audit débit prédéterminé.

3. Procédé de mesure de la pression de la vessie chez un patient selon la revendication 2, dans lequel le débit d'urine réel est mesuré au moyen de la création de signaux électriques représentatifs du débit d'urine réel.

4. Procédé de mesure de la pression de la vessie chez un patient selon l'une quelconque des revendications précédentes, comprenant en outre la détermination de mesures de pression de la vessie durant ladite période de temps basée sur les pressions de brassard mesurées nécessaires pour maintenir ledit débit d'urine prédéterminé durant ladite période de temps.

5. Procédé de mesure de la pression de la vessie chez un patient selon l'une quelconque des revendications précédentes, comprenant le gonflement du brassard (3) pour fermer l'urètre, avant le commencement de l'écoulement d'urine à travers le pénis et après que le brassard (3) ait été attaché autour du pénis du patient, puis le dégonflement du brassard (3) jusqu'à ce que l'écoulement d'urine atteigne ledit débit d'urine prédéterminé.

6. Procédé de mesure de la pression de la vessie chez un patient selon l'une quelconque des revendications 1 à 4, comprenant le gonflement partiel du brassard (3) avant le commencement de l'écoulement d'urine à travers le pénis et après que le brassard (3) ait été attaché autour du pénis du patient, puis le gonflement ou le dégonflement du brassard (3) comme nécessaire, jusqu'à ce que l'écoulement d'urine atteigne ledit débit prédéterminé.

7. Procédé de mesure de la pression de la vessie chez un patient selon l'une quelconque des revendications 1 à 4, comprenant le gonflement du brassard (3) après le commencement de l'écoulement d'urine à travers le pénis et après que le brassard (3) ait été attaché autour du pénis du patient, comme nécessaire, jusqu'à ce que l'écoulement d'urine atteigne ledit débit d'urine prédéterminé.

8. Appareil (1) de mesure de la pression de la vessie chez un patient, comprenant :
(i) un brassard gonflable (3) pour l'attachement autour du pénis du patient ;
(ii) un dispositif produisant une pression pour gonfler le brassard (3) et dégonfler le brassard (3), comme nécessaire ;
**caractérisé par**
(iii) un moyen de contrôle (5) pour maintenir un écoulement d'urine à un débit prédéterminé pour une période de temps durant le vidage de la vessie, au moyen du dégonflement ou du gonflement du brassard (3) sur ladite période de temps, comme nécessaire ; et
(iv) un moyen de mesure (7) pour mesurer les pressions du brassard nécessaires pour maintenir ledit débit d'urine prédéterminé durant ladite période de temps.

9. Appareil (1) de mesure de la pression de la vessie chez un patient selon la revendication 8, comprenant en outre un moyen de mesure d'écoulement (13) pour mesurer le débit d'urine réel et d'entrer le débit mesuré audit moyen de contrôle (5).

10. Appareil (1) pour mesurer la pression de la vessie chez un patient selon la revendication 9, dans lequel ledit moyen de mesure d'écoulement (13) crée des signaux électriques représentatifs du débit d'urine réel.

11. Appareil (1) de mesure de la pression de la vessie chez un patient selon l'une quelconque des revendications 8 à 10, comprenant en outre un moyen d'analyse (9) pour déterminer les mesures de pression de la vessie durant ladite période de temps basé sur les pressions de brassard mesurées nécessaires pour maintenir ledit débit d'urine prédéterminé durant ladite période de temps.
